(19) **Europäisches Patentamt**
**European Patent Office**
**Office européen des brevets**

(11) **EP 2 047 259 B1**

(12) **EUROPEAN PATENT SPECIFICATION**

(45) Date of publication and mention
of the grant of the patent:
**18.04.2012   Bulletin 2012/16**

(51) Int Cl.:
**G01N 33/543** (2006.01)     **C12Q 1/68** (2006.01)

(21) Application number: **06783744.3**

(86) International application number:
**PCT/KR2006/003357**

(22) Date of filing: **25.08.2006**

(87) International publication number:
**WO 2008/007822 (17.01.2008 Gazette 2008/03)**

(54) **BIOSENSOR COMPRISING INTERDIGITATED ELECTRODE SENSOR UNITS**

BIOSENSOR MIT INTERDIGITALEN ELEKTRODENSENSOREINHEITEN

BIODÉTECTEUR À UNITÉS DE DÉTECTION À ÉLECTRODES INTERDIGITÉES

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.07.2006   KR 20060065828**

(43) Date of publication of application:
**15.04.2009   Bulletin 2009/16**

(73) Proprietor: **Korea Research Institute of Standards and Science**
**Daejeon 305-340 (KR)**

(72) Inventors:
• **YUN, Wan-Soo**
  **Daejeon 302-791 (KR)**
• **PARK, Hyung-Ju**
  **Pusan 611-839 (KR)**
• **CHI, Young-Shik**
  **Chungbuk 367-922 (KR)**

(74) Representative: **Albrecht, Thomas**
**Kraus & Weisert**
**Patent- und Rechtsanwälte**
**Thomas-Wimmer-Ring 15**
**80539 München (DE)**

(56) References cited:
WO-A1-03/087798          WO-A1-2007/084077
WO-A2-02/48701           WO-A2-2004/042070
WO-A2-2004/068389        WO-A2-2005/074467
KR-A- 960 024 350        KR-A- 20000 051 475

• **CHIEN-YING TSAI ET AL: "Electrical detection of protein using gold nanoparticles and nanogap electrodes" JAPANESE JOURNAL OF APPLIED PHYSICS, vol. 44, no. 7B, July 2005 (2005-07), pages 5711-5716, XP002557052**

**EP 2 047 259 B1**

**Description**

[Technical Field]

The present invention relates to a biosensor for detecting presence and concentration of various bio-materials including genes and proteins by the electrical method, an interdigitated electrode sensor unit of the biosensor and a method for determining concentration of a bio-material using the biosensor.

[Background Art]

The study of genes and proteins has provided chances to analogize noble biomarkers for diagnosis of diseases. Particularly, in case of the diseases such as cancer, accurate diagnosis in the early stage of development can complete cure of the disease and prevent relapse of the disease. Therefore, studies for development of sensors for early stage detection of biomarkers by antigen-antibody interaction have been actively proceeded. Such sensors can be applied in various fields related to public health, environment, military needs, food and the like and diverse research is in progress.
An optical apparatus can be used to determine the presence of the antigen of a small amount. However, this method has relatively many problems related to time, cost and efforts, though it is widely used. Recently, there are disclosed a method using wavelength change of absorbed light according to the presence of an antigen (Chou et al., (2004) Biosens. Bioelectron. 19, 999-1005) and a method using wavelength change of absorbed light by the change of nano particles having a surface capable of complimentarily binding to an antigen (Alivisatos et al., (2004) Nat. Biotechnol. 22, 47-52, Nam et al., (2003) Science. 301, 1884-1886; US PAT NO. 6, 974, 669).
In recent, researches on electrical detection methods are actively conducted to develop a sensor which is portable, easily usable, cheap and rapid. Such sensors for electrical detection can be readily applied to direct combination with computers, portable equipments, household appliances and the like. There are disclosed methods for detecting concentration of a bio-material from change of the electrical conductivity using a surface-modified carbon nanotube or nanowire (Chen, R. J. et al. (2003) Proc. Natl. Acad. Sci. USA 100, 4984-4989, Zheng, G. et al. (2005) Nat. Biotechnol. 23, 1294-1301; US PAT NO. 6, 781, 166).
However, devices using such a carbon nanotube or nanowire need arrangement techniques to position the nanotube or nanowire in a desired location. Also, since produced devices have different properties according to size and junction resistance of the carbon nanotube and nanowire, they cannot be produced in a mass quantity. Therefore, it is desired to realize a sensor using an device which can be readily integrated and be formed in a desired configuration using patterning (lithography) technology which is used commonly used in the semiconductor process.
The biosensor using nanogap is a technology to meet the above demand and recently attracts interests. According to the devices for detecting bio-material known up-to-date (US PAT NOs. 6,653,653 and 6,824,974), the bio-material connects directly between electrodes. However, they are not suitable for detection of proteins because of high resistance of the biomolecule. Therefore, there is disclosed a method for intermediating nano-particles to electrical conductivity at both ends of the nanogap electrodes (Haguet, V. et al. (2004) Appl. Phys. Lett. 84, 1213-1215). This method is useful in showing effective difference of electrical conductivity. Since this method is based on the general patterning process, it seems to be possible to realize integration of nanogap. However, since the production of nanogap by the general patterning method shows very low yield, it is substantially difficult to form an integrated structure and to provide a reliable result. Moreover, it is impossible to quantitatively analyze the concentration of a subject material.
The present inventors filed a method for preparing a nanogap electrode and a nanogap device prepared by using the method as Korean Patent Application No. 2006-0039528 (published as KR 10 - 0777973 B). The method for preparing a nanogap electrode includes growing a metal by reduction from metal ion in a solution on a surface having a metal pattern formed in a predetermined shape. The nanogap metal electrode prepared according to the method advantageously has a gap of 1 to 50 nm, which is difficult in the prior art.
WO 2004/042070 A2 discloses the electrical detection of DNA hybridisation and specific binding events using a biosensor comprising a plurality of independently-operating interdigitated electrode sensor units integrated on a substrate, wherein the electrodes are formed via classical electrode patterning by semiconductor lithography.
WO 2004/068389 A2 discloses a method of forming a conductive metal region on a substrate using reductive metallisation.
Meanwhile, biosensors have problems of poor reproducibility and accurateness due to a very wide error range according to sensing level of a bio-material in detection of the bio-material. In order to solve these problems, Korean Laid-Open Patent NO. 1996-24350 discloses an interdigitated electrode type biosensor comprising at least two active indication electrodes for generating signals by independently response to a same material existing in a sample, inactive indication electrodes for correcting background signals, a reference electrode and counter electrode. By the interdigitated electrode type biosensor, it is possible to realize accurateness of measurement in only one measurement by providing an average value by processing multiple signals generated from the same subject material in a measurement. However, the disclosed multiple electrode type biosensor comprises a plurality of active indication electrodes having at least one biomaterial selected from enzymes, antigens, antibodies, nucleic acids and molecule receptors immobilized thereon, a reference electrode and a counter electrode is only for averagely measuring size of electrical signals. Also, it is a single electrode type based on the electrochemical method which can be used only in high concentration measurement. Therefore, it has a limit in quantitative analysis such as concentration measurement in a low concentration.

[Disclosure]

[Technical Problem]

Therefore, in order to solve the above-described problems, it is an object of the present invention to provide a biosensor for detecting presence and concentration of various bio-materials such as genes and proteins by the electrical method, an interdigitated electrode sensor unit for forming the biosensor and a method for measuring concentration of a bio-material using the biosensor.

Also, it is another object of the present invention to provide a method for accurate, reliable, qualitative and quantitative measurement of a biomolecule such as antigens both in a low concentration range and in a high concentration range using the biosensor comprising a large area nanogap interdigitated electrode sensor unit manufactured by a simple and high yield production method. It is a further object of the present invention to provide a method for quantitative measurement of the concentration of a bio-material using correlation between concentration and signal probability via statistical measurement of integrated nanogap interdigitated electrode sensor units.

[Technical Solution]

The present invention is directed to a biosensor for detecting presence and concentration of various bio-materials such as genes and proteins by the electrical method, an interdigitated electrode sensor unit for forming the biosensor and a method for measuring concentration of a bio-material using the biosensor.

The biosensor according to the present invention comprises a plurality of independently-operating interdigitated electrode senor units integrated on a substrate, wherein each interdigitated electrode sensor unit comprise: first electrode and second electrode formed interdigitatedly and spaced from each other on the substrate; and a sensor-immobilized biomolecule receptor immobilized on the substrate exposed between the first electrode and the second electrode so that the first electrode can be electrically connected to the second electrode upon binding to a biomolecule and specifically binding to the biomolecule, wherein the first electrode and the second electrode are formed by dipping the substrate having an interdigitated metal pattern thereon in a solution containing metal ion and adding a reducing agent to the solution to grow the metal reduced from the metal ion in the solution on the surface with the metal pattern, and the biomolecule can be analyzed by the number of the interdigitated electrode sensor units electrically connected by the biomolecule captured by the sensor-immobilized biomolecule receptor.

Also, by the biosensor according to the present invention, the biomolecule in a sample solution is quantitatively analyzed by correlation between the concentration of the biomolecule in a sample solution and the ratio of the number of the electrically connected interdigitated electrode sensor unit to the total number of the interdigitated electrode sensor unit.

Particularly, the biosensor according to the present invention comprises interdigitated electrode sensor units provided in an n x m matrix. The interdigitated electrode sensor units are constructed to independently operate so that the interdigitated electrode sensor unit where the biomolecule binds thereto circulates current and does not circulate current where the biomolecule does not bind thereto. Therefore, the concentration of the biomolecule is determined by measuring the number of the current flowing interdigitated electrode sensor units. The number of the interdigitated electrode sensor units circulating current can be readily and automatically measured by a conventional electric circuit layout method.

The electrical conductivity increased by the binding to the biomolecule can be simply determined by measuring resistance of the interdigitated electrode sensor unit.

The voltage and current amount used in the measurement is not particularly limited. However, it is preferable to use a voltage of 100 mV or less, thereby excluding error factors such as electro-migration which can be induced by a strong voltage.

As an example, in the method for determining concentration of a subject material to be analyzed from statistical data of electrical conductivity showing the on-off state of the interdigitated electrode sensor units by integrating the sensor units with m ranks and n columns, the concentration of the subjection material existing in the sample (C) is given by a function of the rate of sensor unit showing the presence of the subject material with respect to the total number of the interdigitated electrode sensor units existing in the biosensor ($N_{tot}$), $P_{on} = N_{on}/N_{tot} = N_{on}/(m \times n)$, where $N_{tot}$ is $m \times n$ and $N_{on}$ is the number of the unit sensors showing the presence of the subject material among the integrated sensor units. Here, when the number of the integrated interdigitated electrode sensor units is increased ($N_{tot}$ is large), the relation between the probability and concentration has a higher reliability. Particularly, the reliability of the concentration measurement is increased when the sensor unit is constructed to have a high sensitivity and a small area to react with a subject material of a small number.

When the rate of the sensor units showing change in the electrical conductivity according to the concentration of the subject antigen existing in the sample increases, it means the increase in the concentration of the subject material. It is possible to determine the concentration of the sample from the rate of the interdigitated electrode sensor units showing change in the electrical conductivity.

The rate ($P_{off}$) of the interdigitated electrode sensor units showing the absence of the subject of the material (off sensor, not showing change in the electrical conductivity) is decreased when the concentration of the subject material is increased. Here, the micro-increment of $P_{off}$ ($dP_{off}$) according to micro-increment of the concentration ($dC$) is proportional to micro-increment of the concentration ($dC$) and the instant rate of the off sensor ($P_{off}$).

$$d\ P_{off}\ =\ -\ k\ \cdot\ P_{off}\ \cdot\ dC\quad \texttt{[k\ is\ a\ proportional\ constant]}$$

From the above equation, the relation between the rate $P_{on}$ of the sensors showing the presence of the subject material (on sensor) and the concentration can be expressed by the following equation.

$$P_{on}\ =\ A\ -\ B\ exp\,(-kC)$$

[A and B refer constants reflecting non-ideal behavior of the sensor which may be caused by several factors such as imperfection of the device, immobilization efficiency of nano-particles and non-specific bonding]

The probability function may vary by various factors such as the structure and arrangement of the biosensor and the interdigitated electrode sensor unit according to the present invention and types of subject materials to be analyzed. Reliability of the concentration measurement can be affected by the size and integration of the device.

The interdigitated electrode biosensor according to the present invention comprises first electrode and second electrode formed to be spaced from and opposed to each other on the substrate, and a sensor-immobilized biomolecule receptor which is immobilized on the substrate exposed between the first electrode and the second electrode so that the first electrode is electrically connected to the second electrode upon binding to a biomolecule to be analyzed and can specifically bind to the biomolecule, in which the first electrode and the second electrode are formed by growing a metal reduced by reduction of metallic ion in a solution on a surface having a predetermined metal pattern formed thereon.

Also, the method for quantitatively analyzing a biomolecule according to the present invention comprises steps of: contacting the biosensor with a sample solution containing the biomolecule to be analyzed so that the biomolecule is captured by a sensor-immobilized biomolecule receptor immobilized on a substrate exposed between first electrode and second electrode of an independently-operating interdigitated electrode sensor unit; contacting the biomolecule bound to the sensor-immobilized biomolecule receptor with a particle-immobilized biomolecule receptor having electrically conductive particle immobilized thereon to bind to the biomolecule; whereby the first electrode and the second electrode are electrically connected by the electrically conductive particle, measuring electrical conductivity of the biosensor; and calculating concentration of the biomolecule in the sample solution from the relation between the concentration and the rate of the sensors showing change of the electrical conductivity before and after contact with the solution.

In another embodiment according the present invention the method comprises the steps of: mixing electrically conductive particle with a sample solution containing a biomolecule to be analyzed for immobilization on the subject biomolecule; contacting the biosensor with the subject biomolecule which have had electrically conductive particle immobilized thereon so that the subject biomolecule is captured by a sensor-immobilized biomolecule receptor immobilized on a substrate exposed between first electrode and second electrode of an independently-operating interdigitated electrode sensor unit; whereby the first electrode and the second electrode are electrically connected by the electrically conductive particle, measuring electrical conductivity of the biosensor; and calculating concentration of the biomolecule in the sample solution from the relation between the concentration and the rate of the sensors showing change of the electrical conductivity before and after contact with the solution. In a further embodiment of the present invention, the method comprises the steps of: contacting the biosensor with a subject biomolecule to be analyzed so that the subject biomolecule is captured by a sensor-immobilized biomolecule receptor immobilized on a substrate exposed between first electrode and second electrode of an independently-operating interdigitated electrode sensor unit; contacting the biosensor with another biomolecule having electrically conductive particle immobilized thereon so that the electrically-conductive-particle-immobilized biomolecule is captured by the sensor-immobilized biomolecule receptor where the subject biomolecule has not been captured; whereby the first electrode and the second electrode are electrically connected by the electrically conductive particle, measuring electrical conductivity of the biosensor; and calculating concentration of the biomolecule in the sample solution from the relation between the concentration and the rate of the sensors showing change of the electrical conductivity before and after contact with the sample solution. The another biomolecule having the electrically conductive particle immobilized thereon includes direct immobilization of the electrically conductive particle on the biomolecule or indirect immobilization of the electrically conductive particle on the biomolecule by the media of biomolecule receptor.

The biosensor according to the present invention includes both the construction, in which the subject biomolecule to be analyzed has electrically conductive particle immobilized thereon so that the first electrode and the second electrode are electrically connected by the electrically conductive particle when the biomolecule binds to the sensor-immobilized biomolecule receptor of the interdigitated electrode sensor unit and the construction, in which the subject biomolecule in the sample solution is captured by and specifically binds to the sensor-immobilized biomolecule receptor of the interdigitated electrode sensor unit and the captured biomolecule then binds to particle-immobilized biomolecule receptor comprising electrically conductive particle immobilized on biomolecule receptor so that the first electrode and the second electrode are electrically connected by the electrically conductive particle.

Here, in the region of the substrate exposed between the two electrodes, the sensor-immobilized biomolecule receptor which can specifically bind to the biomolecule is immobilized. When interdigitated electrodes are used, the area of the substrate exposed by the interdigitated electrode is increased and thus, it is possible to reduce time taken to measure concentration even though the concentration is low.

The sensor-immobilized biomolecule receptor and the particle-immobilized biomolecule receptor may be antibody and the biomolecule may be antigen. Also, as the sensor-immobilized biomolecule receptor, different types of antibodies may be immobilized on the substrate at a predetermined ratio. The antibody may be a material which can selectively react with any antigen to be detected, including genetic materials such as DNA as well as proteins which can undergo antigen-antibody interaction.

When an antibody is used as the sensor-immobilized biomolecule receptor, a monoclonal antibody is preferably used for selectivity of antigen. The reaction conditions such as concentration and time for immobilization on the substrate may follow the known method.

The electrically conductive particle which binds to the biomolecule or the particle-immobilized biomolecule receptor to form a bridge between the first electrode and the second electrode so that the first electrode and the second electrode are electrically connected has a size properly selected according to the gap between the interdigitated electrodes. However, it has preferably a size in the range of 0.5nm to 1$\mu$m and more preferably a size in the range of 1nm to 100nm, considering the nanogap space of the interdigitated electrodes.

The selection of the nano-particle for the particle-immobilized biomolecule receptor is not particularly limited, as long as it has electrical conductivity regardless of properties of semiconductors and metals. However, it is preferably metal such as gold nano-particles. The size of the nano-particle is not particularly limited but is preferably equal to or smaller than the gap space of the nanogap device, more preferably a bit smaller than the gap space.

When an antibody is selected as the particle-immobilized biomolecule receptor, it is preferably polyclonal antibody. The reaction conditions such as concentration and time for binding the electrically conductive particle may follow the known methods.

Meanwhile, the sensor-immobilized biomolecule receptor and the substrate may be fixed by a linker molecule layer such as self assembled monolayer (SAM). Though the selection of the SAM molecule may vary according to circumstances, it is preferably to select a molecule having a functional group such as -CHO, -COOH and the like for bonding to amine group in antibody in most cases.

The substrate having the interdigitated electrode and the sensor-immobilized biomolecule receptor placed thereon may be anyone that can have the biomolecule receptor immobilized and has electrically insulating property. The electrically insulating material is preferably oxides, more preferably silicon oxides.

Preferably, the first electrode and the second electrode have a height greater than the height of the biomolecule receptor immobilized on the substrate considering that the immobilized electrically conductive particle adheres closely between the first electrode and the second electrode to form a bridge for electrical connection. However, it is also possible for the first electrode and the second electrode to have a height smaller than the biomolecule receptor.

The first electrode and the second electrode may be provided with a protein adsorption blocking layer on the surface for preventing adsorption of protein by non-specific binding. The adsorption blocking layer is preferably formed using hydrophilic molecules such as glycol based compounds.

The interdigitated electrode according to the present invention may be patterned by a method selected from lithography, printing and contact printing. Here, the gap between the electrodes may be in the range of 0.5nm to 1$\mu$m. However, considering the size of the biomolecule and bridge formation of electrically conductive particles between the first electrode and the second electrode, the gap between the first electrode and the second electrode is preferably 1nm to 100nm. The electrode can be prepared following the method for preparing nanogap electrode described in Korea Patent Application No. 2006-0039528, filed by the present application.

Thus, the electrode having nanogap according to the present invention is prepared by forming an interdigitated metal pattern by lithography, printing and contact printing and growing a metal reduced by reduction of metallic ion in a solution on a surface having an interdigitated metal pattern formed thereon. More particularly, the electrode is prepared by dipping the substrate having an interdigitated metal pattern formed thereon in a solution containing metal ion and adding a reducing agent to the solution to grow the metal reduced from the metal ion in the solution on the surface with the metal pattern.

Preferably, the metal pattern is selected from Au, Ag, Al, Cu and Pt, considering electrical conductivity and the reducing agent is selected from hydroxyl amine ($H_2NOH$), ascorbic acid, glucose, Rochelle salt (potassium sodium tartrate), formaldehyde and mixtures thereof.

[Description of Drawings]

Further objects and advantages of the invention can be more fully understood from the following detailed description taken in conjunction with the accompanying drawings, in which:

FIG. 1 is a schematic view of the method for detecting a molecule using the interdigitated electrode sensor according to the present invention;

FIG. 2 illustrates the large area interdigitated electrode provided in the interdigitated electrode sensor according to the present invention;

FIG. 3 is a photograph of the interdigitated electrode when antigen is present in a subject sample;

FIG. 4 is a photograph of the interdigitated electrode when antigen is not present in a subject sample;

FIG. 5 shows the electrical characteristics of the biosensor according to the present invention when antigen is present in a subject sample;

FIG. 6 shows the electrical characteristics of the biosensor according to the present invention when antigen is not present in a subject sample;

FIG. 7 is a schematic view showing the method for measuring the concentration from on-off signals of interdigitated electrode sensor units of the biosensor according to the present invention;

FIG. 8 is a graph showing the correlation between the rate of the device showing electrical characteristics variation signal and the concentration of antigen;

FIG. 9 is a schematic view showing another method for quantitatively analyzing a biomolecule according to the present invention; and

FIG. 10 is a schematic view showing correlation between the rate of devices showing changed electrical characteristics in the high concentration range and low concentration range according to (a) the method of FIG. 1 and (b) the method of FIG. 9.

[Mode for Invention]

Hereinafter, a preferred embodiment of the invention will be explained in detail with reference to the appended drawings. However, it would be appreciated by those skilled in the art that changes may be made to these embodiments without departing from the principles and spirit of the invention, the scope of which is defined by the claims .

[Example]

A silicon substrate having an oxide layer thereon was used. An interdigitated gold pattern in the form of a large area IDE (interdigitated electrode) having a gap of about 70 nm - 200 nm was formed by photo and e-beam lithography and then subjected to the gap narrowing process by chemical reduction to prepare 3 biosensors, each biosensor comprising 8 interdigitated electrodes having a gap distance of about 50 nm.

The gap narrowing by chemical reduction was performed by dipping the substrate having the pattern formed thereon 11 mL of 36 $\mu$M $HAuCl_4$ aqueous solution, adding 1 mL of 640 $\mu$M $NH_2OH$ aqueous solution thereto and leaving the solution for 2 minutes at 27 °C for reaction. The above-described procedures were repeated 4 times to grow gold on the interdigitated gold pattern surface. Thus, a nanogap device having interdigitated electrode sensor units integrated thereon was prepared.

Next, a linker molecule layer was formed in the nanogap region where the substrate was exposed between the interdigitated electrodes of the interdigitated electrode sensor unit and antibody which selectively binds to antigen was introduced to form an active surface. The used antibody was anti-PSA which was an antibody which selectively binds to PSA (prostate specific antigen), a marker of prostate cancer.

Firstly, in order to fix the antibody, a molecule layer was formed using the linker molecule in the nanogap region.

Since the nanogap region (silicon oxide layer) was formed a material different from the interdigitated electrode part (gold), it is possible to introduce a selective molecule layer. In order to induce selective binding of the gold nano-particle in the nanogap region, the previously prepared nanogap device was dipped in 1mM solution of 2,5,8,11-tetraoxadocosane-22-thiol for 10 minutes to form ethyleneglycol (EG) group on the gold electrode surface.

Next, antibody was selectively attached to the $SiO_2$ surface existing in the nanogap region. The surface was activated by $O_2$ plasma treatment, reacted with APTES (aminopropyltriethoxysilane) to form a molecule layer having -$NH_2$ terminal and treated with 1% glutaaldehyde buffer solution (0.1M sodium phosphate buffer, pH=7.0) for 30 minutes to bind aldehyde group to the molecule layer formed by using APTES and to form a surface having aldehyde group, which had not participated in the binding to the molecule layer, exposed thereon.

When the aldehyde surface formed on the nanogap surface was dipped in a buffer solution (10 mM PBS buffer, pH=7.4) of monoclonal antibody having a concentration of about 50 mg/mL, the -$NH_2$ group existing in lysine peptide of the monoclonal antibody was effectively reacted with the aldehyde group exposed on the surface, whereby the antibody was fixed on the surface. Then, the unreacted -CHO group was saturated by treatment with 0.1M glycine buffer (pH=8.0) for 30 minutes.

The biosensor thus-obtained was measured for I-V properties in a low voltage range of -100 mV to 100 mV.

The biosensor was contacted with a solution containing PSA to be measured and control solution not containing PSA for about 1 hour and reacted with solution of 10 to 40 nm gold nano-particles having polyclonal anti-PSA thereon at a ratio of 1 to 10 antibodies per nano-particle. Then, in order to minimize probability of error by non-specific adsorption, washing process by buffer solution or de-ionized water was performed. Finally, the nanogap biosensor and the array thereof were dried using nitrogen and the electrical conductivity was measured again. The result of this electrical conductivity measurement was compared with the result of the previous electrical conductivity measurement before the treatment with the subject sample to confirm the change of electrical conductivity. The voltage intensity was set in a range of -100 mV ~ 100 mV to minimize possible error in the measurement such as deformation of antibody and induction of electro migration caused by high voltage.

For quantitative measurement of concentration of a subject sample, a reference curve for the relation between the concentration and the rate of sensors showing electrical conductivity change among the integrated devices was obtained prior to the determination of the concentration of an un-known sample. The relation between concentration and the rate of 'on' sensor needed proportional constant. In a dynamic range of every sensor array, the proportional constant was determined for use in the measurement of that region. It was expected that the dynamic region of the sensor array would vary according to gap-separation and configuration of the nanogap, size of nano-particles, type of antigen-antibody interaction and the surface immobilization method. The reliability of the concentration measurement was raised as the sensitivity of the unit sensor is high and the size of the region is small and as the units are highly integrated.

Now, the preferred embodiment of the present invention will be described in detail with reference to the attached drawings. The following examples are provided as an illustration to deliver the spirit of the present invention to those having ordinary knowledge in the art. Therefore, the present invention is not limited to the examples described below and may be embodied to another form. Also, in the drawings, length and thickness of some layers and regions may be magnified for convenience. Like reference numerals refer to the like devices throughout the specification.

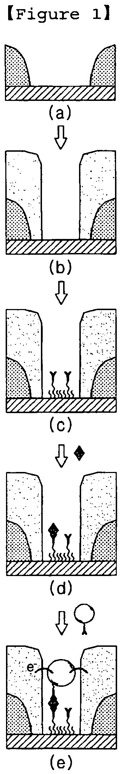

FIG. 1 is a schematic view of the method for detecting a molecule using the interdigitated electrode sensor according to the present invention.

Referring to FIG. 1, the interdigitated electrode biosensor according to the present invention is prepared by forming interdigitated electrode pattern on a substrate so that electrodes are opposed to each other (a). The interdigitated electrode pattern is transformed into nanogap interdigitated electrodes by reduction process (b). Sensor-immobilized biomolecule receptors are immobilized on the substrate exposed between the nanogap interdigitated electrodes through the media of the linker molecule layer (Self Assembled Monolayer) to form a biosensor (c). A sample solution is contacted with the biosensor so that a biomolecule in the sample solution specifically binds to the sensor-immobilized biomolecule receptor (d). Then, particle-immobilized biomolecule receptor (same as the sensor-immobilized biomolecule receptor) having conductive nano-particles immobilized thereon binds to the biomolecule, whereby the first electrode and the second electrode are electrically connected (e).

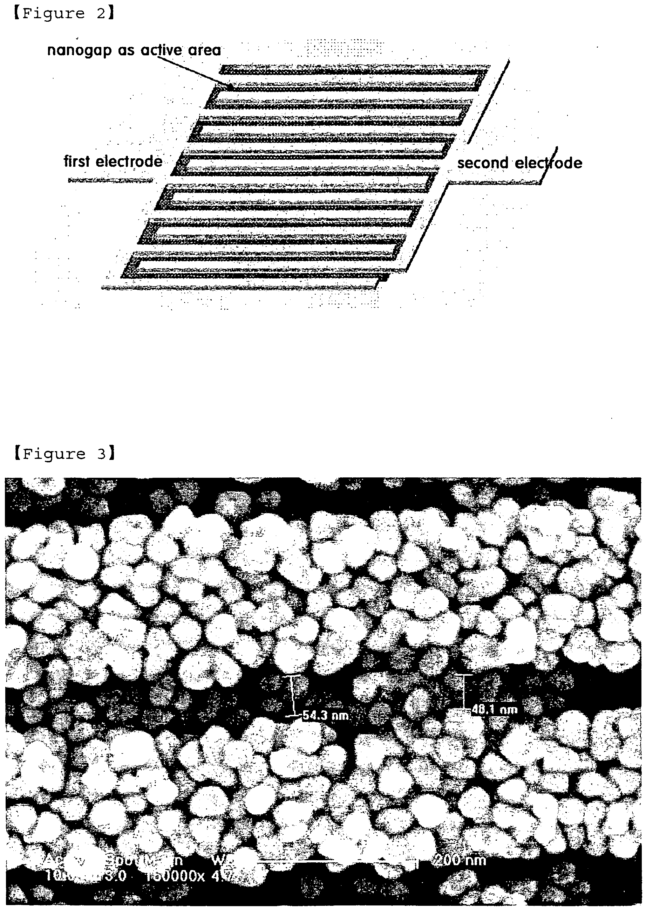

FIG. 2 illustrates the large area interdigitated electrodes engaged with each other according to the present invention, in which the area where the nano-particles may be immobilized is enlarged by increasing the total length of the interdigitated electrode area while maintaining the electrode gap of the interdigitated electrode sensor according to the present invention in a range of 5 nm to several tens of nm, for rapid detection of antigen at a low concentration. In the interdigitated electrode according to the present invention, the first electrode and the second electrode are repeatedly alternated with each other while maintaining a uniform distance and opposed to each other so that the opposed area of the first electrode and the second electrode is increased.

Here, the substrate area exposed between the two electrodes is an area where the sensor-immobilized biomolecule receptor which can specifically bind to the biomolecule is immobilized. When this area is increased, it is possible to reduce the time taken to measure relatively low concentration sample. The method for forming the nanogap device having such structure includes a chemical reduction gap narrowing step to raise yield and reduce the unit cost of production . As the method for increasing the area of the active surface, methods for highly integrating nanogap sensors may be selected.

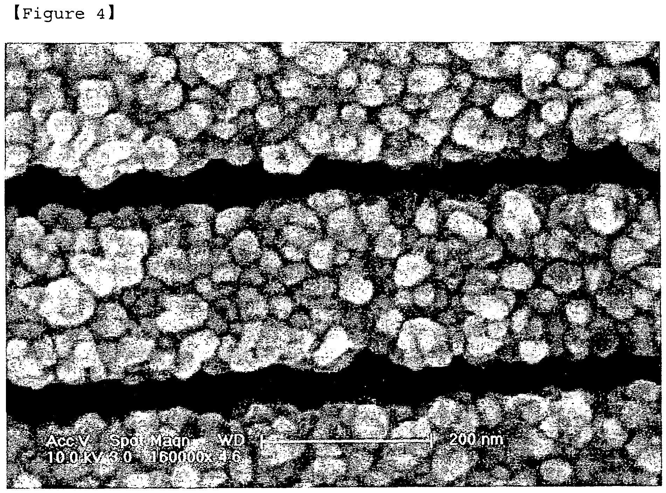

FIG. 3 and FIG. 4 are photographs of the interdigitated electrode when antigen is present in a subject sample and when antigen is not present in a subject sample. In order to confirm the immobilization of the electrically conductive particles, a part of the large area interdigitated electrode sensor unit is magnified. FIG. 3 shows a number of nano-particles captured between nanogap electrodes having a gap of about 50 nm prepared by the gap narrowing process. This is the result that antigen contained in the subject sample is captured by antibody attached onto the surface of the nanogap electrodes and the antigen then binds to antibody having electrically conductive nano-particle, consequently showing nano-particles immobilized between the electrodes. FIG. 4 is an electron microscopic photograph of a sample measured by using the nanogap interdigitated electrode sensor unit and performing the same process as for FIG. 3, except that the sample does not contain antigen which can selectively bind to antibody. Here, it is confirmed that nano-particles are not immobilized since the subject sample does not contain antigen which can selectively bind to antibody.

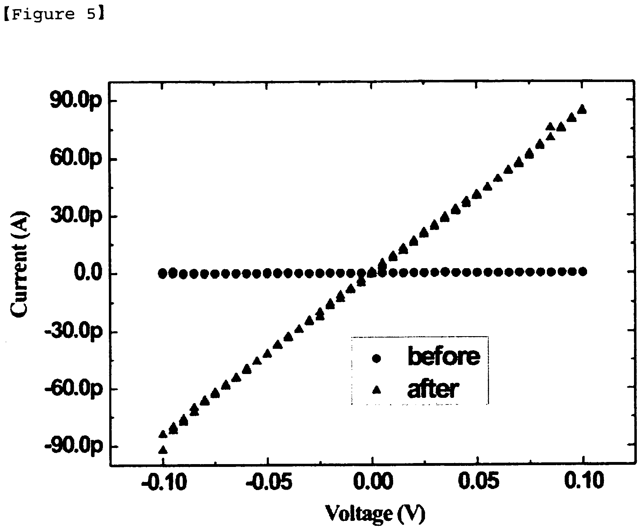

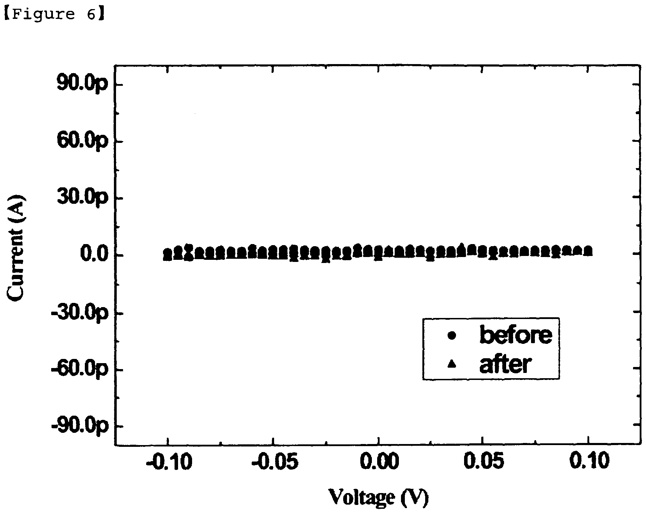

The particle-immobilized biomolecule receptor having electrically conductive particles immobilized in the gap between interdigitated electrodes may induce change in electrical conductivity between two electrodes. FIG. 5 and FIG. 6 show changes in electrical conductivity of the nanogap sensor according to binding of the particle-immobilized biomolecule receptor.

FIG. 5 is an I-V graph showing before and after contact between the biosensor according to the present invention and the biomolecule (antigen) containing sample, in which the electrical conductivity is increased by the particle-immobilized biomolecule receptor binding to the biomolecule. Since the I-V graph after the contact with the biomolecule has a uniform gradient, it can be indirectly confirmed that the electrical conductivity is increased by an electron transport phenomenon by the media of the electrically conductive metallic nano-particles.

FIG. 6 shows the change in the I-V graph before and after contact with the biosensor according to the present invention and a subject sample when the biomolecule is not present in the subject sample. It is shown that the electrical conductivity does not change when the biomolecule is not present in a subject sample, unlike FIG. 5.

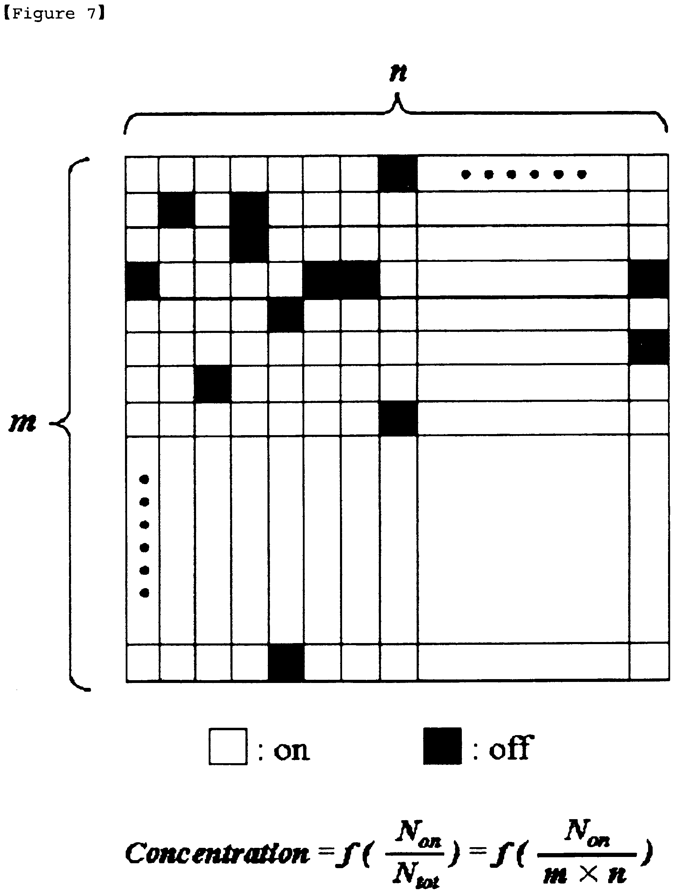

FIG. 7 is a schematic view for description of the method for measuring the concentration of a subject material from statistical data of electrical conductivity showing the on-off state of the interdigitated electrode sensor units by integrating the sensor units with m ranks and n columns.

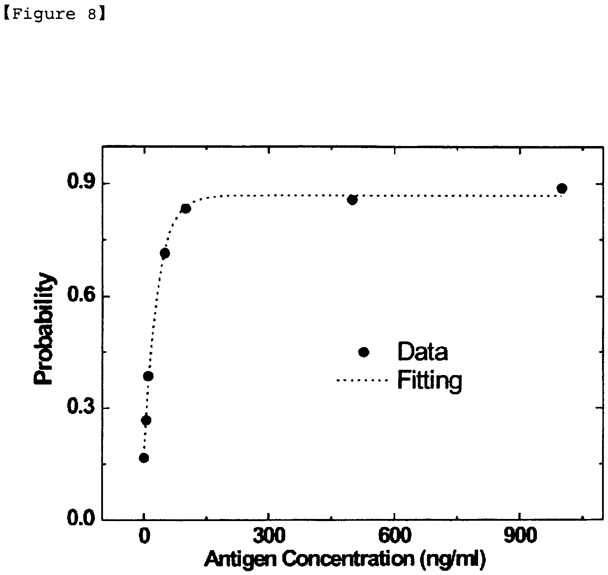

FIG. 8 is a graph showing the correlation between the rate of the device showing electrical characteristics variation signal and the concentration of antigen, in which the measured rate of the interdigitated electrode sensor units causing the change in the electrical conductivity according to the concentration of the antigen on the nanogap biosensor antigen is plotted. The dotted curve is calculated by the following equation. It is confirmed that the experimental result agrees well with the result estimated by the equation.

$$P_{on} = A - B \, exp(-kC)$$

[A and B refer constants reflecting non-ideal behavior of the sensor which may be caused by several factors such as imperfection of the device, immobilization efficiency of nano-particles and non-specific bonding]

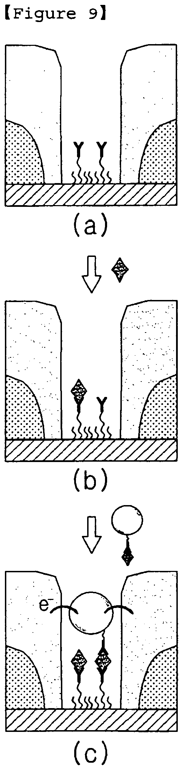

FIG. 9 is a schematic view showing another method for quantitatively analyzing a biomolecule according to the present invention, in which the measurement step is opposed to the method described in FIG. 1. In the following, the method is described in detail with reference to FIG. 9.

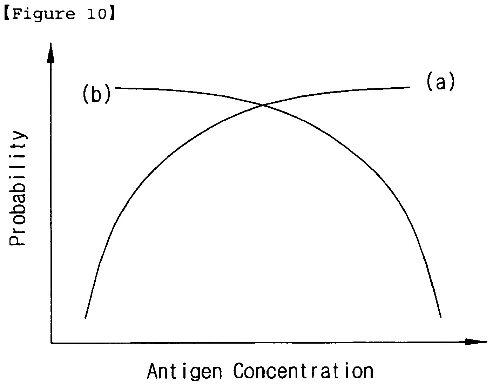

The biosensor according to the present invention is contacted with a sample solution containing a subject biomolecule (a). The biomolecule is captured by sensor-immobilized biomolecule receptors immobilized on a substrate (b). The biosensor is contacted with another biomolecule having electrically conductive particles fixed thereon. The electrically-conductive-particle-fixed biomolecule is captured by sensor-immobilized biomolecule receptor where the subject biomolecule has not been captured. The first electrode and the second electrode are electrically connected by the electrically conductive particle (c). Therefore, the biomolecule in the sample solution can be analyzed by measuring the number of electrically connected interdigitated electrode sensor units or the number of non-connected interdigitated electrode sensor units. The method shown in FIG. 9 can be effectively used in measurement of biomolecule in a high concentration range as shown in FIG. 10 (b). This method can overcome the low sensitivity problem appearing when the biomolecule exists at a high concentration in a quantitative analysis of the biomolecule by the method of FIG. 1 (FIG. 10 (a)).

[Industrial Applicability]

The biosensor according to the present invention can contribute to various fields such as development of material detection technique based on molecular recognition, kit for early diagnosis of diseases, environment monitoring systems, public health and hygiene supervision apparatus, sensor systems for anti-terror and against chemical, biological and radiological warfare, and development of sensor kits. The present invention can provide the highest performance by combination with technique for pre-treatment of reagent such as blood and optimized surface control technique, technique for complex treatment of signals and be conveniently manufactured in a large scale.

Also, according to the present invention, it is possible to detect a small amount of biomolecule in high concentration and low concentration ranges by producing a large area nanogap device by reducing the gap space of the nanogap device and increasing the surface area of the nanogap to shorten the detection time while maintaining detection sensitivity of the device.

**Claims**

1. A biosensor comprising a plurality of independently-operating interdigitated electrode sensor units integrated on a substrate, in which the interdigitated electrode sensor units comprise:

   first electrode and second electrode formed interdigitatedly and spaced from each other on the substrate; and a sensor-immobilized biomolecule receptor immobilized on the substrate exposed between the first electrode and the second electrode so that the first electrode can be electrically connected to the second electrode upon binding to a biomolecule and specifically binding to the biomolecule, wherein the first electrode and the second electrode are formed by dipping the substrate having an interdigitated metal pattern thereon in a solution containing metal ion and adding a reducing agent to the solution to grow the metal reduced from the metal ion in the solution on the surface with the metal pattern, and the biomolecule can be analyzed by the number of the interdigitated electrode sensor units electrically connected by the biomolecule captured by the sensor-immobilized biomolecule receptor.

2. The biosensor according to claim 1, wherein the biomolecule receptor of the sensor-immobilized biomolecule receptor is antibody and the biomolecule is antigen.

3. The biosensor according to claim 2, wherein the sensor-immobilized biomolecule receptor comprises different types of antibodies immobilized on the substrate at a predetermined ratio.

4. The biosensor according to claim 1, wherein the gap between the first electrode and the second electrode is 1 nm to 100 nm.

5. The biosensor according to claim 1, wherein the first electrode and the second electrode are provided with a protein adsorption blocking layer on the surface.

6. The biosensor according to claim 1, wherein the sensor-immobilized biomolecule receptor and the substrate are fixed by a linker molecule layer.

7. The biosensor according to claim 1, wherein the first electrode and the second electrode have a height greater than the height of the biomolecule receptor immobilized on the substrate.

8. The biosensor according to claim 1, wherein the metal pattern is selected from Au, Ag, Al, Cu and Pt.

9. The biosensor according to claim 1, wherein the reduction of the metal ion is performed by adding a reducing agent selected from hydroxyl amine ($H_2NOH$), ascorbic acid, glucose, Rochelle salt (potassium sodium tartrate), formaldehyde and mixtures thereof to the solution.

10. A method for analyzing a biomolecule comprising the steps of:

    contacting the biosensor according to claim 1 with a sample solution containing the biomolecule to be analyzed so that the biomolecule is captured by a sensor-immobilized biomolecule receptor immobilized on a substrate exposed between first electrode and second electrode of an independently-operating interdigitated electrode sensor unit ;

contacting the biomolecule bound to the sensor-immobilized biomolecule receptor with a particle-immobilized biomolecule receptor having electrically conductive particle immobilized thereon to bind to the biomolecule; whereby the first electrode and the second electrode are electrically connected by the electrically conductive particle,

measuring electrical conductivity of the biosensor; and

calculating concentration of the biomolecule in the sample solution from the relation between the concentration and the rate of the sensors showing change of the electrical conductivity before and after contact with the solution.

**11.** A method for analyzing a biomolecule comprising the steps of:

immobilizing electrically conductive particle on the subject biomolecule;

contacting the biosensor according to claim 1 with the subject biomolecule which have had electrically conductive particle immobilized thereon so that the subject biomolecule is captured by a sensor-immobilized biomolecule receptor immobilized on a substrate exposed between first electrode and second electrode of an independently-operating interdigitated electrode sensor unit;

whereby the first electrode and the second electrode are electrically connected by the electrically conductive particle,

measuring electrical conductivity of the biosensor; and

calculating concentration of the biomolecule in the sample solution from the relation between the concentration and the rate of the sensors showing change of the electrical conductivity before and after contact with the solution.

**12.** A method for analyzing a biomolecule comprising the steps of:

contacting the biosensor according to claim 1 with a sample to be analyzed so that the subject biomolecule is captured by a sensor-immobilized biomolecule receptor immobilized on a substrate exposed between first electrode and second electrode of an independently-operating interdigitated electrode sensor unit;

contacting the biosensor with another biomolecule having electrically conductive particle immobilized thereon so that the electrically-conductive-particle-immobilized biomolecule is captured by the sensor-immobilized biomolecule receptor where the subject biomolecule has not been captured;

whereby the first electrode and the second electrode are electrically connected by the electrically conductive particle,

measuring electrical conductivity of the biosensor; and

calculating concentration of the biomolecule in the sample solution from the relation between the concentration and the rate of the sensors showing change of the electrical conductivity before and after contact with the sample solution.

**13.** The method according to any one of claims 10 to 12, wherein the biomolecule in a sample solution is analyzed by correlation between the concentration of the biomolecule in a sample solution and the ratio of the number of the electrically connected interdigitated electrode sensor unit to the total number of the interdigitated electrode sensor unit.

**14.** The method according to any one of claims 10 to 13, wherein the biomolecule receptor of the sensor-immobilized biomolecule receptor and particle-immobilized biomolecule receptor is antibody and the biomolecule is antigen.

**15.** The method according to any one of claims 10 to 14, wherein the electrically conductive particle immobilized on the biomolecule or particle-immobilized biomolecule receptor has a size of 0.5 nm to 1 $\mu$m.

**16.** The method according to claim 15, wherein the electrically conductive particle immobilized on the particle-immobilized biomolecule receptor has a size of 1 nm to 100 nm.

**Patentansprüche**

**1.** Biosensor, umfassend eine Vielzahl von unabhängig arbeitenden interdigitalen Elektrodensensoreinheiten, die an einem Substrat integriert sind, wobei die interdigitalen Elektrodensensoreinheiten umfassen:

erste Elektrode und zweite Elektrode, die interdigital und voneinander beabstandet an dem Substrat ausgebildet sind, und

einen Sensor-immobilisierten Biomolekülrezeptor, der an dem Substrat immobilisiert ist, das zwischen der ersten Elektrode und der zweiten Elektrode exponiert ist, so dass die erste Elektrode bei Bindung an ein Biomolekül und spezifischer Bindung an das Biomolekül elektrisch mit der zweiten Elektrode verbunden werden kann,

wobei die erste Elektrode und die zweite Elektrode gebildet werden, indem das Substrat, das ein interdigitales Metallmuster darauf hat, in eine Lösung, die Metallion enthält, eingetaucht wird und ein Reduktionsmittel zu der Lösung gegeben wird, um das Metall, das aus dem Metallion in der Lösung reduziert wurde, an der Oberfläche mit dem Metallmuster wachsen zu lassen, und das Biomolekül durch eine Reihe der interdigitalen Elektrodensensoreinheiten, die elektrisch durch das Biomolekül, das durch den Sensor-immobilisierten Biomolekülrezeptor eingefangen wurde, elektrisch verbunden werden, analysiert werden kann.

2. Biosensor gemäß Anspruch 1, wobei der Biomolekülrezeptor des Sensor-immobilisierten Biomolekülrezeptors ein Antikörper ist und das Biomolekül ein Antigen ist.

3. Biosensor gemäß Anspruch 2, wobei der Sensorimmobilisierte Biomolekülrezeptor verschiedene Antikörpertypen, die an dem Substrat immobilisiert sind, bei einem vorbestimmten Verhältnis umfasst.

4. Biosensor gemäß Anspruch 1, wobei der Abstand zwischen der ersten Elektrode und der zweiten Elektrode 1 nm bis 100 nm ist.

5. Biosensor gemäß Anspruch 1, wobei die erste Elektrode und die zweite Elektrode mit einer Proteinadsorptionblockierenden Schicht an der Oberfläche versehen sind.

6. Biosensor gemäß Anspruch 1, wobei der Sensorimmobilisierte Biomolekülrezeptor und das Substrat durch eine Linkermolekülschicht fixiert sind.

7. Biosensor gemäß Anspruch 1, wobei die erste Elektrode und die zweite Elektrode eine Höhe haben, die größer ist als die Höhe des Biomolekülrezeptors, der an dem Substrat immobilisiert ist.

8. Biosensor gemäß Anspruch 1, wobei das Metallmuster aus Au, Ag, Al, Cu und Pt ausgewählt ist.

9. Biosensor gemäß Anspruch 1, wobei die Reduktion des Metallions durchgeführt wird, indem ein Reduktionsmittel, ausgewählt aus Hydroxylamin ($H_2NOH$), Ascorbinsäure, Glucose, Rochelle-Salz (Kaliumnatriumtartrat), Formaldehyd und Gemischen davon, zu der Lösung gegeben wird.

10. Verfahren zum Analysieren eines Biomoleküls, umfassend die Schritte:

Inkontaktbringen des Biosensors gemäß Anspruch 1 mit einer Probenlösung, die das zu analysierende Biomolekül enthält, so dass das Biomolekül durch einen Sensor-immobilisierten Biomolekülrezeptor, der an einem Substrat immobilisiert ist, das zwischen erster Elektrode und zweiter Elektrode einer unabhängig arbeitenden interdigitalen Elektrodensensoreinheit exponiert ist, eingefangen wird;

Inkontaktbringen des Biomoleküls, das an den Sensor-immobilisierten Biomolekülrezeptor gebunden ist, mit einem Partikel-immobilisierten Biomolekülrezeptor, der ein elektrisch leitendes Partikel daran immobilisiert hat, um das Biomolekül zu binden;

wobei die erste Elektrode und die zweite Elektrode durch das elektrisch leitende Partikel elektrisch verbunden sind,

Messen der elektrischen Leitfähigkeit des Biosensors und

Errechnen der Konzentration des Biomoleküls in der Probenlösung aus der Beziehung zwischen der Konzentration und der Rate der Sensoren, die eine Änderung der elektrischen Leitfähigkeit vor und nach Kontakt mit der Lösung zeigen.

11. Verfahren zum Analysieren eines Biomoleküls, umfassend die Schritte:

Immobilisieren eines elektrisch leitenden Partikels an dem Subjektbiomolekül;

Inkontaktbringen des Biosensors gemäß Anspruch 1 mit dem Subjektbiomolekül, das ein elektrisch leitendes Partikel daran immobilisiert hat, so dass das Subjektbiomolekül durch einen Sensor-immobilisierten Biomolekülrezeptor eingefangen wird, welcher an einem Substrat immobilisiert ist, das zwischen erster Elektrode und zweiter Elektrode einer unabhängig arbeitenden interdigitalen Elektrodensensoreinheit exponiert ist; wodurch die erste Elektrode und die zweite Elektrode durch das elektrisch leitende Partikel elektrisch verbunden werden,

Messen der elektrischen Leitfähigkeit des Biosensors und

Errechnen der Konzentration des Biomoleküls in der Probenlösung aus der Beziehung zwischen der Konzentration und der Rate der Sensoren, die eine Änderung der elektrischen Leitfähigkeit vor und nach Kontakt mit der Lösung zeigen.

**12.** Verfahren zum Analysieren eines Biomoleküls, umfassend die Schritte:

Inkontaktbringen des Biosensors gemäß Anspruch 1 mit einer zu analysierenden Probe, so dass das Subjektbiomolekül durch einen Sensor-immobilisierten Biomolekülrezeptor eingefangen wird, welcher an einem Substrat immobilisiert ist, das zwischen erster Elektrode und zweiter Elektrode einer unabhängig arbeitenden interdigitalen Elektrodensensoreinheit exponiert ist;

Inkontaktbringen des Biosensors mit einem anderen Biomolekül, das ein elektrisch leitendes Partikel daran immobilisiert hat, so dass das elektrisch leitende Partikel-immobilisierte Biomolekül durch den Sensor-immobilisierten Biomolekülrezeptor, wo das Subjektbiomolekül nicht eingefangen wurde, eingefangen wird;

wodurch die erste Elektrode und die zweite Elektrode durch das elektrisch leitende Partikel elektrisch verbunden werden,

Messen der elektrischen Leitfähigkeit des Biosensors und

Errechnen der Konzentration des Biomoleküls in der Probenlösung aus der Beziehung zwischen der Konzentration und der Rate der Sensoren, die eine Änderung der elektrischen Leitfähigkeit vor und nach Kontakt mit der Probenlösung zeigen.

**13.** Verfahren gemäß einem der Ansprüche 10 bis 12, wobei das Biomolekül in einer Probenlösung durch Korrelation zwischen der Konzentration des Biomoleküls in einer Probenlösung und dem Verhältnis der Anzahl der elektrisch verbundenen interdigitalen Elektrodensensoreinheiten zu der Gesamtzahl der interdigitalen Elektrodensensoreinheiten analysiert wird.

**14.** Verfahren gemäß einem der Ansprüche 10 bis 13, wobei der Biomolekülrezeptor des Sensor-immobilisierten Biomolekülrezeptors und der Partikel-immobilisierte Biomolekülrezeptor ein Antikörper ist und das Biomolekül ein Antigen ist.

**15.** Verfahren gemäß einem der Ansprüche 10 bis 14, wobei das elektrisch leitende Partikel, das an dem Biomolekül immobilisiert ist, oder der Partikel-immobilisierte Biomolekülrezeptor eine Größe von 0,5 nm bis 1 $\mu$m hat.

**16.** Verfahren gemäß Anspruch 15, wobei das elektrisch leitende Partikel, das an dem Partikel-immobilisierten Biomolekülrezeptor immobilisiert ist, eine Größe von 1 nm bis 100 nm hat.

**Revendications**

**1.** Biocapteur comprenant une pluralité d'unités de capteur à électrodes interdigitées fonctionnant indépendamment intégrées sur un substrat, dans lequel les unités de capteur à électrodes interdigitées comprennent :

une première électrode et une deuxième électrode formées interdigitalement et espacées l'une de l'autre sur le substrat ; et

un récepteur de biomolécule immobilisé par capteur immobilisé sur le substrat exposé entre la première électrode et la deuxième électrode de sorte que la première électrode peut être électriquement connectée à la deuxième électrode lors de la liaison à une biomolécule et en particulier de la liaison à la biomolécule,

dans lequel la première électrode et la deuxième électrode sont formées en plongeant le substrat sur lequel se trouve un motif de métal interdigité dans une solution contenant un ion métal et en ajoutant un réducteur à la solution afin de faire croître le métal réduit à partir de l'ion métal dans la solution sur la surface avec le motif de métal, et la biomolécule peut être analysée par le nombre d'unités de capteur à électrodes interdigitées électriquement connectées par la biomolécule capturée par le récepteur de biomolécule immobilisé par capteur.

**2.** Biocapteur selon la revendication 1, dans lequel le récepteur de biomolécule du récepteur de biomolécule immobilisé par capteur est un anticorps et la biomolécule est un antigène.

3. Biocapteur selon la revendication 2, dans lequel le récepteur de biomolécule immobilisé par capteur comprend différents types d'anticorps immobilisés sur le substrat à un rapport prédéterminé.

4. Biocapteur selon la revendication 1, dans lequel l'espace séparant la première électrode et la deuxième électrode est d'1 nm à 100 nm.

5. Biocapteur selon la revendication 1, dans lequel la première électrode et la deuxième électrode sont dotées d'une couche de blocage d'adsorption de protéine sur la surface.

6. Biocapteur selon la revendication 1, dans lequel le récepteur de biomolécule immobilisé par capteur et le substrat sont fixés par une couche de molécule de lieur.

7. Biocapteur selon la revendication 1, dans lequel la première électrode et la deuxième électrode ont une hauteur supérieure à la hauteur du récepteur de biomolécule immobilisé sur le substrat.

8. Biocapteur selon la revendication 1, dans lequel le motif de métal est choisi parmi Au, Ag, Al, Cu et Pt.

9. Biocapteur selon la revendication 1, dans lequel la réduction de l'ion métal est réalisée en ajoutant un agent réducteur choisi parmi l'hydroxylamine ($H_2NOH$), l'acide ascorbique, le glucose, le sel de Rochelle (tartrate de sodium et de potassium), le formaldéhyde et leurs mélanges à la solution.

10. Procédé d'analyse d'une biomolécule comprenant les étapes consistant à :

mettre en contact le biocapteur selon la revendication 1 avec une solution d'échantillon contenant la biomolécule à analyser de sorte que la biomolécule est capturée par un récepteur de biomolécule immobilisé par capteur immobilisé sur un substrat exposé entre la première électrode et la deuxième électrode d'une unité de capteur à électrodes interdigitées fonctionnant indépendamment ;
mettre en contact la biomolécule liée au récepteur de biomolécule immobilisé par capteur avec un récepteur de biomolécule à particule immobilisée sur lequel est immobilisée une particule électriquement conductrice à lier à la biomolécule ;
moyennant quoi la première électrode et la deuxième électrode sont connectées électriquement par la particule électriquement conductrice,
mesurer la conductivité électrique du biocapteur ; et
calculer la concentration de la biomolécule dans la solution d'échantillon d'après la relation entre la concentration et le taux des capteurs indiquant un changement de la conductivité électrique avant et après le contact avec la solution.

11. Procédé d'analyse d'une biomolécule comprenant les étapes consistant à :

immobiliser électriquement une particule conductrice sur la biomolécule sujet ;
mettre en contact le biocapteur selon la revendication 1 et la biomolécule sujet sur laquelle une particule électriquement conductrice a été immobilisée de sorte que la biomolécule sujet est capturée par un récepteur de biomolécule immobilisé par capteur immobilisé sur un substrat exposé entre la première électrode et la deuxième électrode d'une unité de capteur à électrodes interdigitées fonctionnant indépendamment ;
moyennant quoi la première électrode et la deuxième électrode sont électriquement connectées par la particule électriquement conductrice,
mesurer la conductivité électrique du biocapteur ; et
calculer la concentration de la biomolécule dans la solution d'échantillon d'après la relation entre la concentration et le taux des capteurs indiquant un changement de la conductivité électrique avant et après le contact avec la solution.

12. Procédé d'analyse d'une biomolécule comprenant les étapes consistant à :

mettre en contact le biocapteur selon la revendication 1 avec un échantillon à analyser de sorte que la biomolécule sujet est capturée par un récepteur de biomolécule immobilisé par capteur sur un substrat exposé entre la première électrode et la deuxième électrode d'une unité de capteur à électrodes interdigitées fonctionnant indépendamment ;
mettre en contact le biocapteur avec une autre biomolécule sur laquelle une particule électriquement conductrice est immobilisée de sorte que la biomolécule électriquement conductrice à particule immobilisée est capturée par le récepteur de biomolécule immobilisé par capteur là où la biomolécule sujet n'a pas été capturée ; moyennant quoi la première électrode et la deuxième électrode sont électriquement connectées par la particule électriquement conductrice,

mesurer la conductivité électrique du biocapteur ; et

calculer la concentration de la biomolécule dans la solution d'échantillon à partir de la relation entre la concentration et le taux de capteurs affichant un changement de la conductivité électrique avant et après le contact avec la solution d'échantillon.

13. Procédé selon l'une quelconque des revendications 10 à 12, dans lequel la biomolécule dans une solution d'échantillon est analysée par corrélation entre la concentration de la biomolécule dans une solution d'échantillon et le rapport entre le nombre d'unités de capteur à électrodes interdigitées électriquement connectées et le nombre total d'unités de capteur à électrodes interdigitées.

14. Procédé selon l'une quelconque des revendications 10 à 13, dans lequel le récepteur de biomolécule du récepteur de biomolécule immobilisé par capteur et du récepteur de biomolécule immobilisé par particule est un anticorps et la biomolécule est un antigène.

15. Procédé selon l'une quelconque des revendications 10 à 14, dans lequel la particule électriquement conductrice immobilisée sur la biomolécule ou le récepteur de biomolécule immobilisé par particule a une taille de 0,5 nm à 1 $\mu$m.

16. Procédé selon la revendication 15, dans lequel la particule électriquement conductrice immobilisée sur le récepteur de biomolécule immobilisé par particule a une taille de 1 nm à 100 nm.

【Figure 1】

(a)

(b)

(c)

(d)

(e)

【Figure 2】

【Figure 3】

【Figure 4】

【Figure 5】

[Figure 6]

【Figure 7】

$$\square : \text{on} \qquad \blacksquare : \text{off}$$

$$Concentration = f\left(\frac{N_{on}}{N_{tot}}\right) = f\left(\frac{N_{on}}{m \times n}\right)$$

【Figure 8】

【Figure 9】

(a)

(b)

(c)

【Figure 10】

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6974669 B
- US 6781166 B
- US 6653653 B
- US 6824974 B
- KR 20060039528
- KR 100777973 B
- WO 2004042070 A2
- WO 2004068389 A2
- KR 199624350

### Non-patent literature cited in the description

- **CHOU et al.** *Biosens. Bioelectron.,* 2004, vol. 19, 999-1005
- **ALIVISATOS et al.** *Nat. Biotechnol.,* 2004, vol. 22, 47-52
- **NAM et al.** *Science,* 2003, vol. 301, 1884-1886
- **CHEN, R. J. et al.** *Proc. Natl. Acad. Sci. USA,* 2003, vol. 100, 4984-4989
- **ZHENG, G. et al.** *Nat. Biotechnol.,* 2005, vol. 23, 1294-1301
- **HAGUET, V. et al.** *Appl. Phys. Lett.,* 2004, vol. 84, 1213-1215